# EUROPEAN PATENT SPECIFICATION

(11) **EP 0 479 867 B1**
(45) Date of publication and mention of the grant of the patent: **15.05.1996**
(21) Application number: 90910521.5
(22) Date of filing: 26.06.1990
(51) Int. Cl.: A61K 31/56, A61K 31/565, A61K 31/585, A61K 31/57, A61K 31/58

(54) **COMPOSITIONS USEFUL AS CONTRACEPTIVES IN MALES**
ALS MÄNNLICHES KONTRAZEPTIKUM VERWENDBARE ZUSAMMENSETZUNGEN
PREPARATIONS UTILES COMME CONTRACEPTIFS CHEZ L'HOMME

(30) Priority: 27.06.1989 US 371794
(43) Date of publication of application: 15.04.1992
(73) Proprietor: APPLIED MEDICAL RESEARCH, LTD., Fairfax, VA 22030 (US)
(72) Inventor: Cohen, Michael, NL-2243 CM Wassenaar (NL)
(74) Representative: Marchant, James Ian
(86) International application number: NL9000090
(87) International publication number: WO9100095

(56) References cited:
- CHEMICAL ABSTRACTS, Vol. 108, No. 19, 9 May 1988, Columbus, Ohio, US; M.K. VAUGHAN et al.: "Influence of Melatonin on the Testicular Regression Induced by Subcutaneous Testosterone Pellets in Male Rats Kept in Long or Short Photoperiod", see page 110, Abstract No. 161875c
- CHEMICAL ABSTRACTS, Vol. 107, No. 19, 9 November 1987, Columbus, Ohio, US; S. DAKNIN et al.: "Implantation of melatonin in mediobasal hypothalamus enhances the effect", see page 103, abstract no. 169223p
- CHEMICAL ABSTRACTS, Vol. 103, No. 19, 5 March 1986, Columbus, Ohio, US; L.K. MALENDOWICZ: "Testosterone and Melatonin Effects on Adrenal Cortex of Orchiectomized and Pinaelectomized Rats", see page 190, Abstract No. 207248c
- CHEMICAL ABSTRACTS, Vol. 101, No. 5, 30 July 1984, Columbus, Ohio, US; M. RODRIQUEZ et al.: "Effect of Pineal Indoles and Testosterone on Sexual Behavior in the Rat", see page 97, Abstract No. 33680d
- CHEMICAL ABSTRACTS, Vol. 97, No. 23, 6 December 1982, Columbus, Ohio, US; C. I. SISK et al.: "Daily Melatonin Injection Mimic the Short Day-Induced Increase in Negative Feedback Effects of Testosterone on Gonadotropin Secretion in Hamsters", see page 111, Abstract No. 193558x
- CHEMICAL ABSTRACTS, Vol. 87, No. 11, 12 September 1977, Columbus, Ohio, US; R. H. DAVIS: "The effect of melatonin on the response to exogenous testosterone in mice", see page 85, abstract no. 78854x
- CHEMICAL ABSTRACTS, Vol. 74, No. 13, 29 March 1971, Columbus, Ohio, US; L. DEBELJUK: "Effects of Melatonin on Changes Induced by Castration and Testosteron Insexual Structures of Male Rats", see page 46, Abstract No. 61260j

## Description

### Field of the Invention

This invention relates to compositions useful as contraceptive agents in human males. More specifically, the invention relates to compositions comprising a contraceptively effective amount of a combination of melatonin and an androgenic hormone.

### Background of the Invention

The majority of methods for effecting contraception available to humans today are practiced by females. In addition to various physical and chemical barriers to sperm transport that women can use, such as vaginal foams, diaphragms and intrauterine devices a number of oral contraceptives comprising steroidal hormones, typically a combination of a progestogen and an estrogen, have been developed. Men who wish to assume the responsibility for preventing conception have had fewer options. At present, there are only two widely acceptable methods for the human male, the condom and the vasectomy, and there are drawbacks associated with each of these methods. The failure rate with condoms can be high, resulting in unwanted pregnancies. Vasectomies, for all practical purposes, must be considered irreversible, although limited progress has been made in recent years in reversing vasectomies.

A number of efforts have been made to find a safe, effective, hormone- (or other chemical-) based reversible contraceptive for males, but to date they have met with little success. Most known antispermatogenic agents cannot be used in the development of a male contraceptive because they have been found to be nonspecific cytotoxic agents, mutagenic, carcinogenic and/or exhibit deleterious side effects.

For example, androgens, such as testosterone, have been studied. Exogenous testosterone is known to suppress gonadotropin production and thus inhibit both leutinizing hormone (LH) and follicle stimulating hormone (FSH). Normal blood levels of both of these hormones are required for spermatogenesis. In a 1972 study, daily injections of testosterone propionate over a 2-3 month period were shown to produce azoospermia (i.e., a level of sperm production below that required for the fertilization of a female) with no decrease in libido or potency and with a return to normal sperm count within five months after the administration was stopped. Reddy, P.R. and J.M. Rao, Contraception 5:295 (1972). To suppress spermatogenesis, however, serum levels of testosterone had to be greater than those normally found, thereby possibly subjecting the user to adverse effects of excessive testosterone, including increased red cell mass, water retention, increases in serum cholesterol and hypertension. Liver toxicity and carcinogenicity also can be complications of high dosage administration of these compounds. Henderson, J.T., et al. Lancet 1:934 (1971).

In another study, the effectiveness of testosterone oenanthate was found to be dependent upon the frequency of administration, with optimal results occurring with weekly intramuscular injections of 200 mg. See Wu, F.C.W., Clinical Endocrin. 29:443 (1988). At that dosage level and frequency of administration, only 50% of the subjects tested achieved azoospermia, while 40% became oligospermic (i.e., sperm counts under five million/ml) after 10 weeks of treatment. The testosterone administration was found to be ineffective as a contraceptive in the remaining 10% of the men. Recovery of fertility was found to take six months or more. In addition, side effects included weight gain and gynecomastia. Decreasing the frequency of administration even at increased dose levels was found to result in a loss of suppression. Orally effective androgens, such as testosterone undecanoate, given in supraphysiological doses also have been found to be ineffective. See, Nieschlag, E. et al., Contraception 18:607 (1978).

Combinations of androgens and progestogens also have been administered in hopes of seeking more effective gonadotropin suppression by the synergistic action of the two types of hormones. The major finding from this study was that azoospermia could be induced in only a portion of the subjects tested even when relatively high doses were administered. Shearer, S.B., et al. Intl. J. Androl. supp. 2:680 (1978). In another study, a combination of daily oral administration of medroxy-progesterone acetate and percutaneous testosterone cream was found to be ineffective for achieving widespread azoospermia.

Antiandrogens having progestational effects also have been tested as possible contraceptive agents. Although some have been shown to suppress gonadotropins, studies have indicated that their administration can cause a significant loss of libido and sexual potency. Bremner, W.J. and David De Kretser, The New England J. of Med. 295(20):1111 (1976).

A variety of other agents also have been examined as possible contraceptive agents without success, including antineoplastic drugs, nitrofurans, thiophenes and dinitropyrroles. Antineoplastic drugs are known to be toxic to sperm production, but no drugs within this class have been found which inhibit spermatogenesis at a dosage that also is not toxic to other tissues. The other types of compounds listed above were shown in studies to have unacceptable toxicities in other organ systems and have not been used in human trials. Bremner and De Kretser, Id.

Attempts to use estrogen as a contraceptive for males also have failed, as side effects associated with its administration include gynecomastia and libido loss. Marcus, R. and S.G. Koreman Ann. Rev. Med. 27:357 (1976). Another effort to develop a male contraceptive involved the use of gossypol, a phenolic compound extracted from cotton plants, but it was found that this substance caused severe hypokalemia and weakness as well as diarrhea, edema, dyspnea, neuritis and other toxic effects. Lawrence, S.U., A. M. Phar. 21:57 (1981).

Accordingly, it is a principal object of this invention to provide a contraceptive composition for men which will reliably cause azoospermia, is safe, reversible, easily administered and acceptable to potential users. It is a further object of this invention to provide such a contraceptive which will not impair virility, potency or androgen aspects of metabolism.

### Summary of the Invention

The present invention provides use of melatonin for the manufacture of a composition for effecting contraception in a human male by a method which comprises administering a combination of the melatonin and an androgen in a series of daily doses at dose levels sufficient to induce azoospermia. Optionally, the melatonin and androgen can be administered in further combination with a progestogen. In a preferred embodiment, the contraceptives of this invention are administered in oral dosage form.

### Detailed Description of the Invention

Melatonin (N-acetyl-5-methoxytryptamine) is a hormone synthesized and secreted by the pineal gland. The exact role of the hormone has not yet been determined. Exogenous melatonin administration in humans has been studied in conjunction with a hypothesis that an abnormal melatonin rhythm is associated with endogenous depression and for pharmokinetic purposes (Waldhauser, F., Neuroendocrinology 39:307, 313 [1984]) and in connection with sleep-wake rhythms and the phenomenon of "jet-lag" following airplane trips associated with a change in time zones.

Researchers also have investigated the effects of exogenous melatonin administration on the gonads of various animal species. Female rats injected with melatonin at certain times of the day showed an inhibition of ovulation. Studies also showed that the injection of melatonin into female Syrian golden hamsters had an inhibitory effect on the gonads and on ovulation. Similar effects, however, were not shown in other female mammalian species injected with melatonin. Specifically, the administration of melatonin to sheep (Kenneway, D.J. et al., J. Reproductive Fertility 73:859 [1985]) and to primates (Reppert, S.M., et al., Endocrin. 104:295 [1979]) did not result in a direct alteration of their reproductive physiology.

Recently, it has been discovered that pharmacological doses of melatonin administered daily to a human female selectively suppress the normal mid-menstrual cycle surge in LH sufficient to prevent ovulation and thus can be used as an effective contraceptive for human females. See U.S. Patent Number 4,855,305, filed March 23, 1987.

The effects of melatonin administration to male animals also have been studied. The injection of melatonin into male Syrian golden hamsters at certain specific times of the day was found to have an inhibitory effect on the development of the gonads, spermatogenesis and the weight of the testes. In several studies with male rodents, however, melatonin administration was shown to have only a transitory effect on the testes and their ability to produce sperm. If the melatonin was administered on a continuous basis for an extended period of time, the sexual organs in the male rodents became refractory and spermatogenesis resumed, even if the doses of exogenous melatonin were increased. See, for example, Reiter, R.J. Proc. Soc. Exp. Biol. Med. 163:264 (1980); Reiter, R.J. Endocrin. Rev. 1:109 (1980).

The present invention is based on the discovery that pharmacological doses of melatonin administered daily in combination with an androgen to a human male substantially inhibit the production of LH and FSH and thereby decrease sperm production sufficient to achieve azoospermia. The present invention is directed to compositions for effecting contraception in a human male by a method which comprises daily administering to the male of a combination of melatonin and an androgen in dosages which inhibit spermatogenesis sufficiently to induce azoospermia.

In accordance with this invention, melatonin and a selected androgen are administered in dosages sufficient to suppress the user's normal production of leutinizing and follicle stimulating hormones so as to decrease sperm production below that required for the fertilization of a female. Generally, the melatonin is administered in amounts ranging between about 2 mg and about 2000 mg per day. Preferably about 25 mg to about 200 mg melatonin are administered daily. It may be advantageous, although certainly is not required, to initially administer the melatonin at relatively high dosages within this general range until azoospermia is achieved, which can take several weeks. Then, the melatonin can be administered at relatively lower dosages sufficient to maintain azoospermia.

As used herein, the term melatonin also encompasses melatonin analogs which have a contraceptive effect when administered to human males in combination with an androgen. Such melatonin analogs can have the general formula: 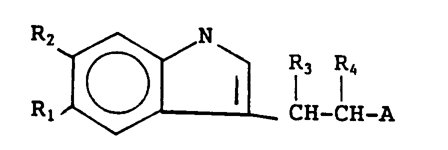
wherein R₁, R₃ and R₄, individually, are hydrogen or an alkyl group having 1 to about 4 carbon atoms, R₂ is selected from hydrogen, hydroxy or an alkoxy group having from 1 to about 4 carbon atoms, and A is selected from the group consisting of -OH or -NH-CO-R₅, wherein R₅ is either hydrogen or an alkyl group having from 1 to about 4 carbon atoms, provided that if A is -NH-CO-R₅, and R₁ and R₅ are both methyl and R₂ is hydrogen, both of R₃ and R₄ are not hydrogen. Preferred compounds are those in which R₂ is hydrogen or methoxy, with hydrogen being most preferred. Melatonin analogs encompassed within this definition include N-acetyl serotonin, N-acetyl, 5-hydroxy, 6-methoxytryptamine, 6-hydroxy-melatonin, 5-hydroxytryptophol and 5-methoxy tryptophol, with N-acetyl serotonin being preferred.

The melatonin is administered in combination with an androgen. Suitable androgens include testosterone, testosterone propionate, testosterone enanthate, testosterone cypionate, methyl testosterone, fluoxymesterone, danazol, dromostanolone propionate, ethylestrenol, methandriol, nandrolone decanoate, nandrolone phenpropionate, oxandrolone, oxymetholone, stanozolol and testolactone. Other male androgens may also be suitable. The androgen generally is administered in daily doses of about 1 mg to about 400 mg. If the androgen used is testosterone, preferred dosages range from about 1.5 mg to about 7.5 mg per day. Preferred dosages of other androgens can be calculated on the basis of their potency relative to that of testosterone. By administering the androgen in combination with melatonin, the dosages of androgen necessary to suppress the levels of sperm production so as to achieve azoospermia are less than those that are needed when the androgen has been tried as the single active component of a male contraceptive. The decreased dosage of androgen is beneficial, as it avoids the problems of depression of libido and potency and the undesirable side effects of excess androgen administration that have been observed in the past. The actual amount of the androgen provided in each daily dose will depend upon the particular androgen chosen, its relative potency, and the method of administration selected. For example, a lesser quantity of a more potent androgen may achieve the same results as a larger quantity of a less potent androgen.

The melatonin and selected androgen conveniently can be combined and administered together, although, if desired, they can be administered separately. The melatonin and androgen can be administered orally, parenterally, by way of depot injection or in the form of an implant. If desired, the melatonin can be administered orally and the androgen can be administered in the form of a depot or vice versa. Administration is most convenient when the hormones are in oral dosage form, such as capsules, tablets, suspensions or solutions. Capsules or tablets are preferred. Capsules can be prepared by mixing the compound with a pharmaceutically-acceptable excipient and then filling gelatin capsules with the mixture in accordance with conventional procedures. Alternatively, the hormones can be mixed with one or more lubricants, such as stearic acid or magnesium stearate, flavor ameliorating agents, disintegrating elements, including potato starch and alginic acid, binders, such as gelatin and corn starch, and/or tablet bases including lactose, corn starch and sucrose, and then pressed into tablets.

As an alternative to oral administration, the hormones can be administered parenterally or in the form of a solid implant. For parenteral administration, the hormones are provided in injectable doses of a solution or suspension of the hormones in a physiologically acceptable diluent with a pharmaceutical carrier. The carrier can comprise water or an oil and optionally can contain a surfactant or other pharmaceutically acceptable adjuvant. Suitable oils include those of animal, vegetable, petroleum or synthetic origin, including peanut, soybean, corn, sesame, castor and mineral oil. Preferred liquid carriers include water, saline, aqueous sugar solutions and glycols such as propylene glycol or polyethylene glycol. Desirably, such injectable formulations are provided for intramuscular depot injections.

The hormones also can be administered in the form of an implant, which is formulated such that it will provide a sustained release of the melatonin over time. To make the implant, the hormones can be compressed into small cylinders and placed inside a physiologically acceptable shell material such as a biodegradable or porous polymer in accordance with conventional implant technology.

In a preferred embodiment of this invention, the contraceptive compositions are administered in oral dosage form, preferably in the form of pills or capsules. The pills or capsules can be packaged in any manner suitable for delivery and use. Conveniently, they can be packaged in the form of a pharmaceutical kit or package similar to those conventionally used for oral contraceptives for women in which the daily unit dosage forms are provided or arranged in a contiguous, sequential order which will enable the man taking the pills to take the proper formulation on any given day. Suitable kits or packages include the conventional bubble package containing individual bubbles for a selected number of days in a sheet of flexible plastic. The bubbles are sealed by a sheet of plastic which can break and release a pill when the bubble is pressed. On the first day of medication, the first pill in the sequence is removed from its individual slot and taken. The next pill in the sequence is taken the next day and so on thereafter until the dispenser is empty. Appropriate notations or instructions can be placed on the dispensing kit to guide or instruct the user in the proper use of oral contraceptives.

In a preferred embodiment of this invention, the melatonin and androgen are administered in further combination with a progestogen. Efficiency of spermatogenesis is dependent upon GnRH and the LH pulses. The GnRH is affected by the administration of progestogens. Melatonin amplifies the effect of the progestogens such that smaller quantities of progestogen can be used to impact spermatogenesis than otherwise would be sufficient. Any progestationally active compound is suitable for use as the progestogen component of the present invention. Suitable progestogens include progesterone and derivatives thereof. The presently preferred progestogen is norethindrone (i.e., 19-nor-17α-ethynyl-17β-hydroxy-4-androsten-3-one) and norgestrel (13β-ethyl-17α-ethynyl-17βhydroxygon-4-en-3-one). Other progestogens include the chlormadinone-acetate (6-chloro-17-hydroxy-pregna-4,6-diene-3,20-dione acetate), norethynodrel (17α-ethynyl-17-hydroxy-estr-5(10)-en), medroxyprogesterone acetate (17α-acetoxy-6α-methyl-pregn-4-ene-3,20-dione), megestrol acetate (17α-acetoxy-6-methyl-pregna-4,6-diene-3,20-dione), lynestrenol (17α-ethynyl-17β-hydroxy-estr-4-ene), quingestrone (3-cyclopentyloxy-pregna-3,5-diene-20-one), norethindrone acetate (17β-acetoxy-17α-ethnyl-estr-4-en-3-on), ethynodiol acetate (3β,17β-diacetoxy-17α-ethynyl-estr-4-ene), dimethisterone [17β-hydroxy-6α-methyl-17(-1-propynyl)-androst-4-en-3-one], and levonorgestrel.

The progestogen is administered in the range of about 10 µg to about 1 mg per day, preferably in the range of about 30 µg to about 150 µg per day. As with the androgen component of the contraceptives of this invention, the actual amount of progestogen provided in each daily dosage depends upon the particular progestogen chosen, its relative potency and the method of administration selected, with lower doses typically needed for administration of an implant or injection than for oral administration.

In this embodiment of the invention, the three active components conveniently can be combined and administered together, although they also can be administered separately.

Alternatively, if desired, an estrogen can be administered in place of the progestogen. Suitable estrogens include ethinyl estradiol (i.e. 17α-ethynyl-3,17β-dihydroxy-estra-1,3,5(10)-triene) and mestranol (17α-ethynyl-17β-hydroxy-3-methoxy-estra-1,3,5(10)triene). Other suitable estrogens include estradiol (3,17β-dihydroxy-estra-1,3,5(10-triene), estradiol(3,-16α,17β-trihydroxy-estera-1,3,5(10)-triene, estrone (3,hydroxy-estra-1,3,5(10)-triene-17-one), diethylstilbestrol, quinestradiol (3-cyclopentyloxy-16α,17β-dihydroxy-estra-1,3,5-(10)-triene) and estrone sulfate. Typically, they can be administered in dosages ranging from about 2 µg to about 100 µg per day; preferably in dosages ranging from about 10 µg to about 50 µg per day.

A number of regimens are suitable for administering the combination of melatonin, androgen and progestogen (or estrogen). In one embodiment, the melatonin and androgen are administered every day on a continuous basis. If desired, the dosage can be relatively high until azoospermia has been achieved, then lesser quantities may be sufficient to maintain the condition. For example, in one regimen, a combination of about 500 to about 1200 mg of melatonin and about 100 mg to about 300 mg androgen is administered by means of an intramuscular depot injection on a weekly basis until azoospermia is achieved. If a progestogen also is included in the formulation, it typically can be provided in dosages of about 500 µg to about 1 mg. Then, to maintain the condition, it may be possible to either increase the length of time between injections or decrease the concentration of each hormone in the preparation. If desired, once azoospermia has been achieved, the hormones can be administered conveniently on a maintenance basis in oral form.

In an alternative embodiment, the melatonin and androgen are administered solely in oral dosage form. With daily oral administration, lesser amounts of the hormones typically are needed than when administration is by injection. In one regimen, the melatonin is administered in dosages of about 50 mg to about 150 mg per day and the androgen is administered in dosages of about 20 mg to about 50 mg per day on a continuous daily basis. If the regimen further comprises a progestogen, the progestogen typically is administered in dosages of about 100 µg to about 400 µg per day. Other useful regimens can be determined using routine experimentation by those with ordinary skill in the art, taking into consideration a particular patient's physiology, the method of administration he prefers and the specific androgen selected for administration.

As noted above, it also has been discovered that the administration of melatonin and an androgen in the amounts disclosed above can be effective in preventing prostate cancer and other forms of cancer that affect the sexual organs and accessory sexual organs of the human male. This discovery provides an important benefit to human males who take the compositions of this invention as a contraceptive. The melatonin and androgen are administered in dosages sufficient to prevent or significantly reduce the risks of getting cancer. Generally, the melatonin and androgen are administered in the general dosages and regimens set forth above.

The present invention is further described and illustrated by the following examples, which are provided for informational purposes and are not to be construed as unduly limiting the scope of the invention.

### Example I

A twenty-three year old male is administered a depot injection of 1000 mg melatonin and 200 mg testosterone oenanthate on a weekly basis. The patient becomes azoospermic and contraceptive efficacy is achieved. The injections are continued to maintain azoospermia.

### Example II

A thirty-five year old man is administered a pill on a daily basis which comprises 75 mg/day melatonin, 40 mg/day androgen and 1 mg/day progestogen. Contraceptive effects are achieved after 4 weeks.

### Example III

A combination of 7.5 mg melatonin, 30 mg 19-nortestosterone, and 100 µg progestogen is administered in a depot preparation intramuscularly. Azoospermia is achieved after 30 days. At the end of four years of monthly injections the medication is stopped and spermatogenesis resumes.

### Example IV

A combination of 200 mg testosterone oenanthate and 5000 mg melatonin is administered to a thirty two year old man on a weekly basis by means of a depot injection until azoospermia is achieved (one month). The patient now continues medication with a combination of 75 mg melatonin and 40 mg testosterone oenanthate in tablet form on a daily basis to maintain azoospermia.

### Example V

A twenty nine year old man is medicated with a depot injection of melatonin (5000 mg) and testosterone propionate (200 mg) on a weekly basis for three weeks until azoospermia is achieved. The patient then continues a daily oral preparation of methyl testosterone (10 mg) and melatonin (75 mg) for continuous administration to maintain azoospermia and effective contraception.

### Example VI

A forty-one year old man seeking contraceptive medication is started on a combination pill daily intake of methyl testosterone and melatonin in dosage ratios of 25 mg and 75 mg, respectively. He is checked on a weekly basis until azoospermia has been induced. During the induction period the patient uses alternative forms of contraception. When azoospermia has been determined (after a period of two to four weeks) he continues the daily medication and relies on the medication as a contraceptive.

### Example VII

A twenty-five year old man seeks contraceptive therapy but does not want to take daily medication or frequent injections. He is given a subdermal implant which comprises a mixture of testosterone propionate, melatonin and norethindrone for slow release. The dosages are calculated to produce supraphysiological levels of melatonin and testosterone and therapeutic levels of norethindrone. Medication capsules are kept in place (subdermally) for six years. When the client wishes to resume fertility the capsules are removed by means of a simple surgical incision and capsule withdrawal.

### Example VIII

A twenty six year old man is started on oral medication with a combination pill containing 150 mg melatonin, 10 mg methyl-testosterone and 150 µg norethindrone. This combination pill is taken twice a day by the patient, once in the morning and once at night before retiring. The pills are taken on a continuous basis.

### Example IX

A twenty seven year old man is prescribed an oral contraceptive which contains 75 mg melatonin, 25 mg methyl testosterone and 300 µg norethindrone per day. The combination pill is administered on a daily basis and sperm checks are instituted on a weekly basis. Azoospermia is achieved during the sixth week of medication. The medication is continued on a daily basis as an effective contraceptive.

## Claims

1. Use of melatonin or a melatonin analog having azoospermia inducing effect for the manufacture of a composition for effecting contraception in a human male by a method which comprises administering a combination of the melatonin or melatonin analog and an androgen in a series of daily doses at dose levels sufficient to induce azoospermia.

2. Use according to Claim 1, wherein the dosage level of melatonin or melatonin analog is from 2 mg to 2000 mg per day of administration, preferably 25 mg to 200 mg per day of administration.

3. Use according to Claim 1 or 2, wherein the androgen is testosterone, testosterone propionate, testosterone enanthate, testosterone cypionate, methyl testosterone, fluoxymesterone, danazol, dromostanolone propionate, ethylestrenol, methandriol, nandrolone decanoate, nandrolone phenpropionate, oxandrolone, oxymetholone, stanozolol or testolactone.

4. Use according to Claim 3, wherein the dosage of androgen is from 1 mg to 400 mg per day of administration, preferably 1.5 mg to 7.5 mg per day of administration.

5. Use according to any of Claims 1 to 4 wherein the method comprises administering a combination of melatonin or a melatonin analog, an androgen and a progestogen, in a series of daily doses at dose levels sufficient to induce azoospermia.

6. Use according to Claim 5, wherein the progestogen is norethindrone, norgestrel, chlormadinone-acetate, norethynodrel, medroxyprogesterone acetate, megestrol acetate, lynestrenol, quingestrone, norethindrone acetate, ethynodiol acetate, dimethisterone, or levonorgestrel.

7. Use according to Claim 6, wherein the dosage of progestogen is from 10 µg to 1 mg per day of administration, preferably 30 µg to 150 µg per day of administration.

8. Use according to any of Claims 1 to 4 wherein the method comprises administering a combination of melatonin or a melatonin analog, an androgen and an estrogen, in a series of doses at dose levels sufficient to induce azoospermia.

9. Use according to Claim 8, wherein the estrogen is ethinyl estradio, mestranol, estradiol, estradiol estrone, diethylstilbestrol, quinestradiol or estrone sulfate.

10. Use according to Claim 9, wherein the dosage level of estrogen ranges from 2 µg to 100 µg per day of administration.

11. Use according to any of Claims 1 to 10, wherein the method of administration is oral, by parenteral injection in a physiologically suitable carrier, or by subcutaneous implant.

12. Use according to any of Claims 1 to 11 wherein the melatonin or melatonin analog component of the combination is melatonin itself.

13. Use according to any of Claims 1 to 11 wherein the melatonin or melatonin analog component of the combination is a melatonin analog of the general formula: 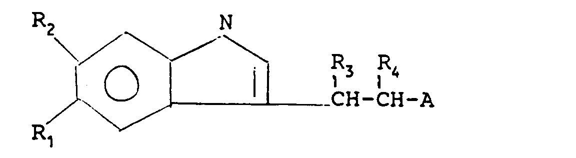 wherein R₁, R₃ and R₄, individually, are hydrogen or an alkyl group having 1 to 4 carbon atoms, R₂ is hydrogen, hydroxy or an alkoxy group having from 1 to 4 carbon atoms, and A is -OH or or -NH-CO-R₅ wherein R₅ is hydrogen or an alkyl group having from 1 to 4 carbon atoms, provided that when A is -NH-CO-R₅, R₁ and R₅ are both methyl and R₂ is hydrogen, then R₃ and R₄ are not both hydrogen.

14. A composition for effecting contraception in a human male which comprises a contraceptively effective combination of melatonin or a melatonin analog having an azoospermia inducing effect and an androgen.

15. A composition according to Claim 14, wherein the androgen is testosterone, testosterone propionate, testosterone enanthate, testosterone cypionate, methyl testosterone, fluoxymesterone, danazol, dromostanolone propionate, ethylestrenol, methandriol, nandrolone decanoate, nandrolone phenpropionate, oxandrolone, oxymetholone, stanozolol or testolactone.

16. A composition according to Claim 14 or 15, which is provided in daily dosage form, wherein each dose comprises from 25 mg to 200 mg of melatonin or melatonin analog and from 1 mg to 400 mg of an androgen.

17. A composition according to any of Claims 14 to 16, wherein the melatonin or melatonin analog and androgen are provided in a solution or suspension of a physiologically acceptable diluent with a pharmaceutical carrier.

18. A composition according to any of Claims 14 to 17 which comprises a contraceptively effective combination of melatonin or a melatonin analog, an androgen and a progestogen.

19. A composition according to Claim 18, wherein the progestogen is norethindrone, norgestrel, chlormadinone-acetate, norethynodrel, medroxyprogesterone acetate, megestrol acetate, lynestrenol, quingestrone, norethindrone acetate, ethynodiol acetate, dimethisterone, and levonorgestrel.

20. A composition according to Claim 18 or 19 which is provided in daily dosage form, wherein each dose comprises from 25 mg to 200 mg of melatonin, or melatonin analog, from 1 mg to 400 mg of an androgen, and from 10 µg to 1 mg of a progestogen.

21. A composition according to any of Claims 14 to 17 which comprises a contraceptively effective combination of melatonin or a melatonin analog, an androgen and an estrogen.

22. A composition according to Claim 21 wherein the estrogen is ethinyl estradiol mestranol, estradiol, estradiol estrone, diethylstilbestrol, quinestradiol or estrone sulfate.

23. A composition according to Claim 21 or 22 which is provided in daily dosage form, wherein each dose comprises from 25 mg to 200 mg of melatonin or melatonin analog, from 1 mg to 400 mg of an androgen, and from 1 µg to 100 µg of an estrogen.

24. A composition according to any of Claims 14 to 23 wherein the melatonin or melatonin analog component of the combination is melatonin itself.

25. A composition according to any of Claims 14 to 23, wherein the melatonin or melatonin analog component of the combination is melatonin analog of the general formula 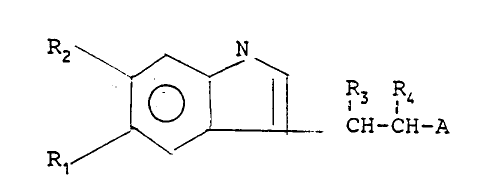 wherein R₁, R₃ and R₄, individually, are hydrogen or an alkyl group having 1 to 4 carbon atoms, R₂ is hydrogen, hydroxy or an alkoxy group having from 1 to 4 carbon atoms, and A is -OH or or -NH-CO-R₅ wherein R₅ is hydrogen or an alkyl group having from 1 to 4 carbon atoms, provided that when A is -NH-CO-R₅, R₁ and R₅ are both methyl and R₂ is hydrogen, then R₃ and R₄ are not both hydrogen.

26. A dispensing package having oral contraceptives in unit dosage forms therein, said unit dosage forms being adapted for oral administration of one unit dosage form daily, and each unit dosage form comprising 2 mg to 2000 mg melatonin or a melatonin analog having an azoospermin inducing effect and 1 mg to 400 mg of an androgen, said package comprising a unitary member having individual storage pods formed therein, said storage pods corresponding to the number of days during which said unit dosage forms are to be administered.

## Patentansprüche

1. Verwendung von Melatonin oder einem Melatonin-Analogon, das einen Azoospermie-induzierenden Effekt besitzt, zur Herstellung einer Zusammensetzung zur Bewirkung der Empfängnisverhütung in einem männlichen Menschen durch ein Verfahren, das die Verabreichung einer Kombination aus dem Melatonin oder Melatonin-Analogon und einem Androgen in einer Reihe von täglichen Dosen in einer Dosismenge, die zur Induzierung der Azoospermie ausreicht, umfaßt.

2. Verwendung gemäß Anspruch 1, worin die Dosierungsmenge an Melatonin oder Melatonin-Analogon 2 mg bis 2000 mg pro Verabreichungstag beträgt, bevorzugterweise 25 mg bis 200 mg pro Verabreichungstag.

3. Verwendung gemäß Anspruch 1 oder 2, worin das Androgen Testosteron, Testosteronpropionat, Testosteronenanthat, Testosteroncypionat, Methyltestosteron, Fluoxymesteron, Danazol, Dromostanolonpropionat, Ethylestrenol, Methandriol, Nandrolondecanoat, Nandrolonphenpropionat, Oxandrolon, Oxymetholon, Stanzozolol oder Testolacton ist.

4. Verwendung gemäß Anspruch 3, worin die Androgen-Dosierung 1 mg bis 400 mg pro Verabreichungstag beträgt, bevorzugterweise 1,5 mg bis 7,5 mg pro Verabreichungstag.

5. Verwendung gemäß mindestens einem der Ansprüche 1 bis 4, worin das Verfahren die Verabreichung einer Kombination aus Melatonin oder einem Melatonin-Analogon, einem Androgen und einem Progestogen in einer Reihe von täglichen Dosen in einer Dosismenge, die zur Induzierung von Azoospermie ausreicht, umfaßt.

6. Verwendung gemäß Anspruch 5, worin das Progestogen Norethindron, Norgestrel, Chlormadinon-acetat, Norethynodrel, Medroxyprogesteronacetat, Megestrolacetat, Lynestrenol, Chingestron, Norethindronacetat, Ethynodiolacetat, Dimethisteron oder Levonorgestrel ist.

7. Verwendung gemäß Anspruch 6, worin die Dosierung des Progestogens 10 µg bis 1 mg pro Verabreichungstag beträgt, bevorzugterweise 30 µg bis 150 µg pro Verabreichungstag.

8. Verwendung gemäß mindestens einem der Ansprüche 1 bis 4, worin das Verfahren die Verabreichung einer Kombination aus Melatonin oder einem Melatonin-Analogon, einem Androgen und einem Östrogen in einer Reihe von Dosen in Dosismengen, die zur Induzierung von Azoospermie ausreichen, umfaßt.

9. Verwendung gemäß Anspruch 8, worin das Östrogen Ethinylöstradioi, Mestranol, Östradiol, Östradiolöstron, Diethyistilbestrol, Chinöstradiol oder Östronsulfat ist.

10. Verwendung gemäß Anspruch 9, worin die Dosismenge an Östrogen im Bereich von 2 µg bis 100 µg pro Verabreichungstag liegt.

11. Verwendung gemäß mindestens einem der Ansprüche 1 bis 10, worin das Verabreichungsverfahren oral, parenterale Injektion in einem physiologisch annehmbaren Träger oder subkutane Implantation ist.

12. Verwendung gemäß mindestens einem der Ansprüche 1 bis 11, worin die Melatonin- oder Melatonin-Analogon-Komponente der Kombination Melatonin selber ist.

13. Verwendung gemäß mindestens einem der Ansprüche 1 bis 11, worin die Melatonin- oder Melatonin-Analogon-Komponente der Kombination ein Melatonin-Analogon der allgemeinen Formel 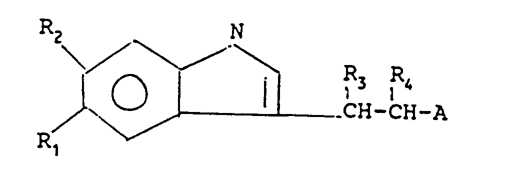 ist, worin R₁, R₃ und R₄ unabhängig voneinander Wasserstoff oder eine Alkyl-Gruppe mit 1 bis 4 Kohlenstoffatomen sind, R₂ ist Wasserstoff, eine Hydroxy-Gruppe oder eine Alkoxy-Gruppe mit 1 bis 4 Kohlenstoffatomen, und A ist -OH oder -NH-CO-R₅, worin R₅ Wasserstoff oder eine Alkyl-Gruppe mit 1 bis 4 Kohlenstoffatomen ist, vorausgesetzt daß, wenn A -NH-CO-R₅, R₁ und R₅ jeweils Methyl und R₂ Wasserstoff ist, R₃ und R₄ nicht beide Wasserstoff sind.

14. Zusammensetzung zur Bewirkung der Empfängnisverhütung in einem männlichen Menschen, die eine empfängnisverhütend wirksame Kombination aus Melatonin oder einem Melatonin-Analogon, das eine Azoospermie-induzierende Wirkung besitzt, und einem Androgen umfaßt.

15. Zusammensetzung gemäß Anspruch 14, worin das Androgen Testosteron, Testosteronpropionat, Testosteronenanthat, Testosteroncypionat, Methyltestosteron, Fluoxymesteron, Danazol, Dromostanolonpropionat, Ethylestrenol, Methandriol, Nandrolondecanoat, Nandrolonphenpropionat, Oxandrolon, Oxymetholon, Stanozolol oder Testolacton ist.

16. Zusammensetzung gemäß Anspruch 14 oder 15, die in Tagesdosisform bereitgestellt wird, worin jede Dosis 25 bis 200 mg Melatonin oder Melatonin-Analogon und 1 bis 400 mg eines Androgens umfaßt.

17. Zusammensetzung gemäß mindestens einem der Ansprüche 14 bis 16, worin das Melatonin oder Melatonin-Analogon und das Androgen in einer Lösung oder Suspension eines physiologisch annehmbaren Verdünnungsmittels mit einem pharmazeutischen Träger bereitgestellt wird.

18. Zusammensetzung gemäß mindestens einem der Ansprüche 14 bis 17, die eine empfängnisverhütend wirksame Kombination aus Melatonin oder einem Melatonin-Analogon, einem Androgen und einem Progestogen umfaßt.

19. Zusammensetzung gemäß Anspruch 18, worin das Progestogen Norethindron, Norgestrel, Chlormadinonacetat, Norethynodrel, Medroxyprogesteronacetat, Megestrolacetat, Lynestrenol, Chingestron, Norethindronacetat, Ethynodiolacetat, Dimethisteron und Levonorgestrel ist.

20. Zusammensetzung gemäß Anspruch 18 oder 19, die in Tagesdosisform bereitgestellt wird, worin jede Dosis 25 bis 200 mg Melatonin oder Melatonin-Analogon, 1 bis 400 mg eines Androgens und 10 µg bis 1 mg eines Progestogens umfaßt.

21. Zusammensetzung gemäß mindestens einem der Ansprüche 14 bis 17, die eine empfängnisverhütend wirksame Kombination aus Melatonin oder einem Melatonin-Analogon, einem Androgen und einem Östrogen umfaßt.

22. Zusammensetzung gemäß Anspruch 21, worin das Östrogen Ethinylöstradiol, Mestranol, Östradiol, Östradiolöstron, Diethylstilbestrol, Chinöstradiol oder Östronsulfat ist.

23. Zusammensetzung gemäß Anspruch 21 oder 22, die in Tagesdosisform bereitgestellt wird, worin jede Dosis 25 bis 200 mg Melatonin oder Melatonin-Analogon, 1 bis 400 mg eines Androgens und 1 bis 100 µg eines Östrogens umfaßt.

24. Zusammensetzung gemäß mindestens einem der Ansprüche 14 bis 23, worin die Melatonin- oder Melatonin-Analogon-Komponente der Kombination Melatonin selber ist.

25. Zusammensetzung gemäß mindestens einem der Ansprüche 14 bis 23, worin die Melatonin- oder Melatonin-Anologon-Komponente der Kombination ein Melatonin-Anologon der allgemeinen Formel 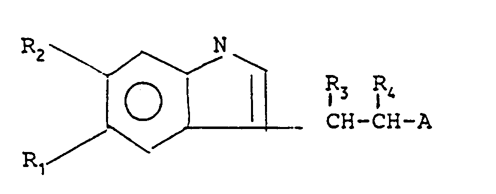 ist, worin R₁, R₃ und R₄ unabhängig voneinander Wasserstoff oder eine Alkyl-Gruppe mit 1 bis 4 Kohlenstoffatomen sind, R₂ ist Wasserstoff, eine Hydroxy-Gruppe oder eine Alkoxy-Gruppe mit 1 bis 4 Kohlenstoffatomen, und A ist -OH oder -NH-CO-R₅, worin R₅ Wasserstoff oder eine Alkyl-Gruppe mit 1 bis 4 Kohlenstoffatomen ist, vorausgesetzt daß, wenn A -NH-CO-R₅, R₁ und R₅ jeweils Methyl und R₂ Wasserstoff ist, R₃ und R₄ nicht beide Wasserstoff sind.

26. Ausgabepackung, in der sich orale Verhütungsmittel in Einheitsdosierungsform befinden, die Einheitsdosierungsformen sind angepaßt an orale Verabreichung einer Einheitsdosierungsform pro Tag, und jede Einheitsdosierungsform umfaßt 2 bis 2000 mg Melatonin oder ein Melatonin-Analogon, das eine Azoospermie induzierende Wirkung besitzt, und 1 bis 400 mg eines Androgens, die Packung umfaßt ein Matrix-Element, in dem individuelle Aufbewahrungsschalen ausgebildet sind, die Aufbewahrungsschalen entsprechen der Anzahl der Tage während derer die Einheitsdosierungsformen verabreicht werden sollen.

## Revendications

1. Utilisation de la mélatonine ou d'un analogue de la mélatonine ayant un effet inducteur de l'azoospermie pour la fabrication d'une composition destinée à réaliser la contraception chez l'homme par un procédé qui comprend l'administration d'une combinaison de la mélatonine ou d'un analogue de la mélatonine et d'un androgène dans une série de doses journalières à des dosages suffisants pour induire l'azoospermie.

2. Utilisation selon la revendication 1, dans laquelle la dose de mélatonine ou d'un analogue de la mélatonine est comprise entre 2mg et 2000mg par jour d'administration, de préférence entre 25mg et 200mg par jour d'administration.

3. Utilisation selon la revendication 1 ou 2, dans laquelle l'androgène est la testostérone, le propionate de testostérone, l'énanthate de testostérone; le cypionate de testostérone, la testostérone méthylique, le fluoxymestérone, le danazol, le propionate de dromostanolone, l'éthylestrénol, le méthandriol, le décanoate de nandrolone, le phènpropionate de nandrolone, l'oxandrolone, l'oxymétholone, le stanozolol ou le testolactone.

4. Utilisation selon la revendication 3, dans laquelle la dose d'androgène est comprise entre 1 mg et 400mg par jour d'administration, de préférence entre 1,5mg et 7,5mg par jour d'administration.

5. Utilisation selon l'une quelconque des revendications 1 à 4 dans laquelle le procédé comprend l'administration d'une combinaison de mélatonine ou d'un analogue de la mélatonine, d'un androgène ou d'un progestogène, dans une série de doses journalières à des dosages suffisants pour induire l'azoospermie.

6. Utilisation selon la revendication 5, dans laquelle le progestogène est le noréthindrone, le norgestrel, le chlormadinone-acétate, le noréthynodrel, l'acétate de médroxyprogestérone, l'acétate de mégestrol, le lynestrénol, le quingestrone, l'acétate de noréthindrone, l'acétate d'éthynodiol, le diméthistérone, ou le lévonorgestrel.

7. Utilisation selon la revendication 6, dans laquelle la dose de progestogène est comprise entre 10µg et 1mg par jour d'administration, de préférence entre 30µg et 150µg par jour d'administration.

8. Utilisation selon l'une quelconque des revendications 1 à 4 dans laquelle le procédé comprend l'administration d'une combinaison de mélatonine ou d'un analogue de la mélatonine, d'un androgène et d'un oestrogène, dans une série de doses à des dosages suffisants pour induire l'azoospermie.

9. Utilisation selon la revendication 8, dans laquelle l'oestrogène est l'éthyloestradiol, le mestranol, l'oestradiol, l'oestradiol oestrone, le diéthylstilbestrol, le quinestradiol ou le sulfate d'oestrone.

10. Utilisation selon la revendication 9, dans laquelle la dose d'oestrogène est comprise entre 2µg et 100µg par jour d'administration.

11. Utilisation selon l'une quelconque des revendications 1 à 10, dans laquelle le procédé d'administration se fait par voie orale, par injection parentérale dans un porteur physiologiquement acceptable, ou par implant sous-cutané.

12. Utilisation selon l'une quelconque des revendications 1 à 11 dans laquelle le composé mélatonine ou analogue de la mélatonine de la combinaison est la mélatonine elle-même.

13. Utilisation selon l'une quelconque des revendications 1 à 11 dans laquelle le composé mélatonine ou analogue de la mélatonine de la combinaison est un analogue de la mélatonine de formule générale: 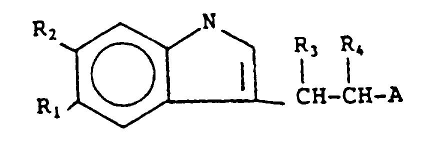 où R₁, R₃ et R₄, individuellement, sont l'hydrogène ou un groupe alcoyle ayant 1 à 4 atomes de carbone, R₂ est choisi parmi l'hydrogène, un groupe hydroxyle ou un groupe alcoxyle ayant 1 à 4 atomes de carbone, et A est -OH ou -NH-CO-R₅ où R₅ est l'hydrogène ou un groupe alcoyle ayant 1 à 4 atomes de carbone, pourvu que si A est -NH-CO-R₅, R₁ et R₅ sont tous les deux un méthyle et R₂ est l'hydrogène, à la fois R₃ et R₄ ne soient alors pas l'hydrogène.

14. Composition destinée à réaliser la contraception chez l'homme qui comprend une combinaison efficace du point de vue contraceptif de la mélatonine ou d'un analogue de la mélatonine ayant un effet inducteur de l'azoospermie et un androgène.

15. Composition selon la revendication 14, dans laquelle l'androgène est la testostérone, le propionate de testostérone, l'énanthate de testostérone, le cypionate de testostérone, le testostérone méthylique, le fluoxymestérone, le danazol, le propionate de dromostanolone, l'éthylestrénol, le méthandriol, le décanoate de nandrolone, le phénpropionate de nandrolone, l'oxandrolone, l'oxymétholone, le stanozolol et le testolactone.

16. Composition selon la revendication 14 ou 15, laquelle est fournie sous forme de doses journalières, dans laquelle chaque dose comprend 25mg à 200mg de mélatonine ou d'un analogue de la mélatonine et 1mg à 400mg d'un androgène.

17. Composition selon l'une quelconque des revendications 14 à 16, dans laquelle la mélatonine ou un analogue de la mélatonine et un androgène sont fournis dans une solution ou une suspension d'un diluant acceptable du point de vue physiologique avec un porteur pharmaceutique.

18. Composition selon l'une quelconque des revendications 14 à 17 qui comprend une combinaison efficace du point de vue contraceptif de mélatonine ou d'un analogue de la mélatonine, d'un androgène et d'un progestogène.

19. Composition selon la revendication 18, dans laquelle le progestogène est le noréthindrone, le norgestrel, le chlormadinone-acétate, le noréthynodrel, l'acétate de médroxyprogestérone, l'acétate de mégestrol, le lynestrol, le quingestrone, l'acétate de noréthindrone, l'acétate d'éthynodiol, le diméthistérone et le lévonorgestrel.

20. Composition selon la revendication 18 ou 19 qui est fournie sous la forme de doses journalières, dans laquelle chaque dose comprend 25mg à 200mg de mélatonine, ou d'un analogue de la mélatonine, 1mg à 400mg d'un androgène, et 10µg à 1mg d'un progestogène.

21. Composition selon l'une quelconque des revendications 14 à 17 qui comprend une combinaison efficace du point de vue contraceptif de mélatonine ou d'un analogue de la mélatonine, d'un androgène et d'un oestrogène.

22. Composition selon la revendication 21 dans laquelle l'oestrogène est l'éthinyloestradiol, le mestranol, l'oestradiol, l'oestradiol oestrone, le diéthylstilbestrol, le quinestradiol ou le sulfate d'oestrone.

23. Composition selon la revendication 21 ou 22 qui est fournie sous la forme de doses journalières, dans laquelle chaque dose comprend 25mg à 200mg de mélatonine ou d'un analogue de la mélatonine, 1mg à 400mg d'un androgène, et 1µg à 100µg d'un oestrogène.

24. Composition selon l'une quelconque des revendications 14 à 23 dans laquelle le composé mélatonine ou analogue de la mélatonine de la combinaison est la mélatonine elle-même.

25. Composition selon l'une quelconque des revendications 14 à 23, dans laquelle le composé mélatonine ou analogue de la mélatonine de la combinaison est un analogue de la mélatonine de formule générale 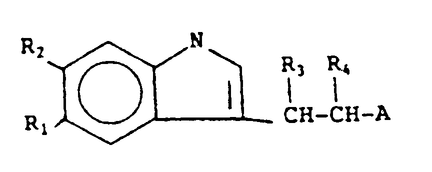 où R₁, R₃ et R₄, individuellement, sont l'hydrogène ou un groupe alcoyle ayant 1 à 4 atomes de carbone, R₂ est choisi parmi l'hydrogène, un groupe hydroxyle ou un groupe alcoxyle ayant 1 à 4 atomes de carbone, et A est -OH ou -NH-CO-R₅ où R₅ est l'hydrogène ou un groupe alcoyle ayant 1 à 4 atomes de carbone, pourvu que si A est -NH-CO-R₅, R₁ et R₅ sont tous les deux un méthyle et R₂ est l'hydrogène, à la fois R₃ et R₄ ne soient pas alors l'hydrogène.

26. Distributeur comportant des contraceptifs oraux sous forme de doses unitaires, lesdites doses unitaires étant prévues pour l'administration par voie orale d'une dose unitaire journalière, et chaque dose unitaire comprenant 2mg à 2000mg de mélatonine ou d'un analogue de la mélatonine ayant un effet inducteur de l'azoospermie et 1mg à 400mg d'un androgène, ledit distributeur comprenant un élément unitaire comportant des cavités de stockage individuelles, lesdites cavités de stockage correspondant au nombre de jours pendant lesquels lesdites doses unitaires doivent être administrées.
